(19) Europäisches Patentamt
European Patent Office
Office européen des brevets

(11) Numéro de publication: **0 089 908**
**B1**

(12) # FASCICULE DE BREVET EUROPEEN

(45) Date de publication du fascicule du brevet: **02.05.85**

(51) Int. Cl.⁴: **C 07 C 51/56**, C 07 C 53/12

(21) Numéro de dépôt: **83420052.9**

(22) Date de dépôt: **22.03.83**

(54) **Nouveau procédé de préparation d'anhydrides d'acides carboxyliques.**

(30) Priorité: **23.03.82 FR 8205268**

(43) Date de publication de la demande: **28.09.83 Bulletin 83/39**

(45) Mention de la délivrance du brevet: **02.05.85 Bulletin 85/18**

(84) Etats contractants désignés: **AT BE CH DE FR GB IT LI LU NL SE**

(56) Documents cités: **FR - A - 1 313 360**

(73) Titulaire: **RHONE-POULENC CHIMIE DE BASE, 25, quai Paul Doumer, F-92408 Courbevoie (FR)**

(72) Inventeur: **Gallucci, Jacques, 2, Boulevard Général de Gaulle, F-69600 Oullins (FR)**
Inventeur: **Gauthier-Lafaye, Jean, 21, rue Duguesclin, F-69006 Lyon (FR)**
Inventeur: **Leconte, Philippe, 45, rue Duquesne, F-69006 Lyon (FR)**
Inventeur: **Perron, Robert, La Pecolière, F-69390 Charly (FR)**

(74) Mandataire: **Varnière-Grange, Monique et al, RHONE-POULENC RECHERCHES Service Brevets Chimie et Polymères Centre de Recherches de Saint-Fons 85, rue des Frères Perret B.P. 62, F-69192 St-Fons Cédex (FR)**

**EP 0 089 908 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention a pour objet un nouveau procédé de préparation d'anhydrides d'acides carboxyliques.

La présente invention a plus particulièrement pour objet un procédé de préparation d'anhydrides d'acides carboxyliques par une nouvelle réaction de carbonylation mettant en œuvre un catalyseur à base de cobalt, cette réaction pouvant être effectuée dans des conditions de température et de pression relativement douces.

Il est bien connu que l'anhydride acétique peut être obtenu par réaction du monoxyde de carbone sur l'acétate de méthyle en présence d'un catalyseur choisi parmi les complexes du cobalt de formule générale:

$$(R_4A)_2CoX_4$$

dans laquelle X représente un atome de brome ou d'iode, A un atome d'azote ou de phosphore et R un radical alkyle inférieur, par exemple (cf. le brevet américain N° 2730546). Toutefois, l'efficacité de ce type de catalyseurs reste faible, bien que des pressions dans la gamme de 200 à 650 bar et des températures de l'ordre de 180 à 190° C soient mises en œuvre.

Par ailleurs, le brevet français N° 1313360 décrit diverses réactions de carbonylation mettant en œuvre un sel de l'hydrure de tétracarbonylcobalt, en milieu basique, qui peuvent toutes être représentées par le schéma réactionnel:

$$RX + CO + BH \rightarrow RCOB + HX$$

dans lequel R peut être un radical alkyle, X peut être un atome d'halogène, et BH représente une molécule renfermant un atome d'hydrogène mobile (eau, alcool, phénol, mercaptan, etc.). Ces réactions sont en général conduites à la pression atmosphérique ou sous quelques dizaines de bars, et à des températures de l'ordre de 25 à 50° C.

Il a maintenant été trouvé qu'il est possible de préparer des anhydrides d'acides carboxyliques par une nouvelle réaction de carbonylation catalysée par des composés du cobalt, cette réaction pouvant être conduite dans des conditions de pression et de température nettement plus douces que celles jusqu'alors requises.

La présente invention a donc pour objet un procédé de préparation d'anhydrides d'acides carboxyliques de formule:

$$RCO-O-COR' \qquad (I)$$

dans laquelle R est un radical alkyle linéaire ayant au maximum 4 atomes de carbone et R' est un radical alkyle linéaire ou ramifié ayant de 1 à 12 atomes de carbone, un radical phényle ($C_6H_5-$), un radical $C_6H_5-C_xH_{2x}-$ ou un radical $C_xH_{2x+1}-C_6H_4-$, x étant un entier compris entre 1 et 6, R et R' pouvant par ailleurs être identiques ou différents, par réaction dans une phase liquide pratiquement anhydre renfermant un solvant organique aprotique

a) d'un composé de formule:

$$RX \qquad (II)$$

dans laquelle R a la signification précédente et X représente un atome d'iode ou un groupement $R''-SO_3-$, R'' étant un radical alkyle, aryle ou aralkyle ayant au maximum 10 atomes de carbone,

b) d'un carboxylate de formule:

$$(R'COO^-)_nA^{n+} \qquad (III)$$

dans laquelle R' a la signification précédente, n est un entier égal à 1, 2, 3 ou 4 et $A^{n+}$ est un cation choisi dans le groupe constitué par les cations des métaux alcalins, les cations des métaux alcalino-terreux, les cations $N^+ R^1 R^2 R^3 R^4$ et les cations $P^+ R^1 R^2 R^3 R^4$ dans lesquels $R^1$ à $R^4$, identiques ou différents, ont la signification donnée précédemment pour R', $R^1$ à $R^4$ pouvant en outre être choisis parmi les radicaux cycloalkyles comportant de 3 à 8 atomes de carbone, le cation $Mn^{++}$, le cation $Zn^{++}$, les cations des métaux du groupe des lanthanides et les cations des métaux du groupe des actinides, et

c) du monoxyde de carbone,

en présence d'un sel de l'hydrure de tétracarbonylcobalt [hydrure ayant pour formule $HCo(CO)_4$], à une température comprise entre 0 et 200° C, sous une pression inférieure ou égale à 600 bar.

Le procédé selon la présente invention qui peut être représenté par le schéma réactionnel suivant:

$$RX + CO + 1/n\,(R'COO^-)_nA^{n+} \rightarrow RCO-O-COR' \qquad (1)$$

est mis en œuvre dans une phase liquide pratiquement anhydre. Par phase liquide pratiquement anhydre, la titulaire entend un milieu liquide dans les conditions de la réaction, qui renferme le moins d'eau possible compte tenu des diverses contraintes industrielles. On notera toutefois que la présence de quantités mineures d'eau, telles que celles susceptibles d'être apportées dans la charge ou l'alimentation par des réactifs de qualité technique, est tolérable, mais non souhaitable.

Cette phase liquide peut être considérée comme étant pratiquement homogène, c'est-à-dire que dans les conditions de réaction le processus de carbonylation se déroule dans un milieu dans lequel une partie prépondérante des différentes matières de départ et du catalyseur se trouve en solution.

Comme indiqué ci-avant, la phase liquide pratiquement anhydre renferme un solvant organique aprotique. De nombreuses classes de tels solvants conviennent à la mise en œuvre de la présente invention.

Plus spécifiquement, conviennent à la mise en œuvre du présent procédé les solvants couramment appelés solvants aprotiques dipolaires qui correspondent au type N° 4 dans le schéma de classification des solvants selon Bronsted figurant dans «The Chemistry of Nonaqueous Solvents», vol. III, pp. 13-16, édité par J.J. Lagowski, 1970 (Academic Press), ainsi que les anydrides d'acides carboxyliques, les éthers, ou les mélanges de ces différents composés.

Ainsi, le solvant en question peut être l'anhydride d'acide carboxylique produit ou tout autre anhydride d'acide carboxylique de formule I

précitée. L'anhydride acétique est un solvant plus particulièrement approprié à la mise en pratique du présent procédé.

Le solvant en question peut être également choisi parmi les éthers cycliques tels que le dioxanne, le tétrahydrofuranne, les éthers macrocycliques plus couramment appelés éthers couronnes ou bien parmi les éthers alkyliques de polyéthylèneglycols de formule:

$$R^5-O-(CH_2-CH_2-O)_y-R^6 \quad (IV)$$

dans laquelle $R^5$ et $R^6$, identiques ou différents, ont la signification donnée précédemment pour R, y étant un entier compris entre 1 et 8. A titre d'exemples d'éthers alkyliques de polyéthylèneglycols utilisables dans le cadre du présent procédé, on peut citer l'éther diméthylique de l'éthylèneglycol (couramment appelé glyme), l'éther diméthylique du diéthylèneglycol, l'éther diéthylique du diéthylèneglycol, l'éther dibutylique du diéthylèneglycol, l'éther éthylique butylique du diéthylèneglycol, l'éther diméthylique du triéthylèneglycol et l'éther diméthylique du tétraéthylèneglycol (tétraglyme).

Le solvant peut aussi être choisi, de manière appropriée, parmi les sulfones de formule:

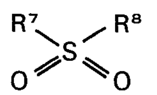

(V)

dans laquelle $R^7$ et $R^8$, identiques ou différents, ont la signification donnée précédemment pour R'; $R^7$ et $R^8$ peuvent en outre former ensemble un radical unique divalent — alkylène ou alcénylène — comportant de 3 à 6 atomes de carbone (par exemple un radical tétraméthylène ou hexaméthylène) et le cas échéant 1 ou 2 doubles liaisons éthyléniques, ledit radical pouvant comporter 1 à 3 substituants alkyles ayant de 1 à 4 atomes de carbone. Conviennent à la mise en œuvre de l'invention, notamment les dialkylsulfones, composés de formule (V) ci-avant dans laquelle $R^7$ et $R^8$ identiques sont des radicaux alkyles linéaires, et plus particulièrement la tétraméthylènesulfone, la méthyl-3-tétraméthylènesulfone, la diméthyl-2,4-tétraméthylènesulfone et leurs mélanges.

Le solvant peut, par ailleurs, tout aussi bien être choisi parmi les amides d'acides carboxyliques de formule:

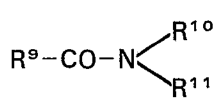

(VI)

dans laquelle $R^9$, $R^{10}$ et $R^{11}$, identiques ou différents, ont la signification donnée précédemment pour R'; deux des radicaux $R^9$, $R^{10}$, $R^{11}$ peuvent par ailleurs constituer ensemble un radical unique divalent $-(CH_2)_z-$, z étant un entier compris entre 3 et 12; $R^9$ peut en outre représenter un atome d'hydrogène ou un radical de formule:

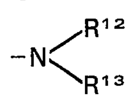

(VII)

dans laquelle $R^{12}$ et $R^{13}$, identiques ou différents, représentent des radicaux alkyles ayant au maximum 4 atomes de carbone. A titre d'exemples de tels solvants on peut citer:
la tétraméthylurée,
le N,N-diméthylformamide,
le N,N-diméthylacétamide,
le N,N-diéthylacétamide,
le N,N-dicyclohexylacétamide,
le N,N-diméthylpropionamide,
le N,N-diéthylpropionamide,
le N,N-diéthyl-n-butyramide,
le N,N-diméthylbenzamide,
le N,N-dicyclohexylbenzamide,
le N,N-diéthyl-m-toluamide,
la N-acétylpyrrolidine,
la N-acétylpipéridine,
la N-(n-butyryl)pipéridine,
la N-méthylpyrrolidone-2,
la N-éthylpyrrolidone-2,
la N-méthylpipéridone-2,
le N-méthyl-ε-caprolactame.

Le solvant peut également être choisi parmi les nitriles de formule:

$$R^{14}-CN \quad (VIII)$$

dans laquelle $R^{14}$ a la signification donnée précédemment pour R'. A titre d'exemples de tels composés, on peut citer l'acétonitrile et le benzonitrile.

Le choix parmi ces nombreuses catégories de solvants sera avant tout orienté par des considérations d'ordre pratique, telles que la solubilité des matières premières utilisées et la facilité de séparation du produit dans le solvant considéré.

On opère, avantageusement, avec un solvant choisi parmi l'anhydride acétique, la tétraméthylènesulfone et la N-méthylpyrrolidone-2.

Ainsi, selon la présente invention, on utilise comme matière de départ un composé de formule RX dans laquelle R est un radical alkyle linéaire ayant au maximum 4 atomes de carbone et X représente un atome d'iode ou un groupement $R''-SO_3-$, R'' étant un radical alkyle, aryle ou aralkyle ayant au maximum 10 atomes de carbone. A titre d'exemples de tels réactifs, on peut citer l'iodure de méthyle, l'iodure de n-butyle, l'ester méthylique de l'acide méthanesulfonique, l'ester méthylique de l'acide éthanesulfonique, l'ester éthylique de l'acide éthanesulfonique, l'ester méthylique de l'acide benzènesulfonique, l'ester méthylique de l'acide p-toluènesulfonique, l'ester n-butylique de l'acide benzènesulfonique, l'ester n-butylique de l'acide p-toluènesulfonique et l'ester méthylique de l'acide phénylméthanesulfonique.

Conviennent plus particulièrement à la mise en œuvre du présent procédé les iodures d'alkyle et les esters alkyliques de l'acide p-toluènesulfonique. L'iodure de méthyle est un réactif plus particulièrement approprié dans le cadre de cette technique.

Une autre matière de départ utilisée lors de la mise en œuvre du présent procédé est un carboxylate de formule:

$$(R'COO^-)_nA^{n+} \quad (III)$$

dans laquelle R', $A^{n+}$ et n ont été définis précédemment.

Une première catégorie de carboxylates utilisables dans le cadre du présent procédé est constituée par les carboxylates alcalins et les carboxylates alcalino-terreux. A titre d'exemples de tels composés, on peut citer les acétates de lithium, de sodium, de potassium, de calcium et de magnésium, le benzoate de sodium et le phénylacétate de sodium.

Une deuxième catégorie de carboxylates utilisables dans le cadre du présent procédé est constituée par les carboxylates des métaux des groupes des lanthanides et des actinides. A titre d'exemples de tels composés, on peut citer les acétates de lanthane, de cérium, de thorium.

Une troisième catégorie de carboxylates utilisables dans le cadre du présent procédé est constituée par les carboxylates d'ammonium quaternaire et les carboxylates de phosphonium quaternaire dont les cations sont respectivement représentés par les formules $N^+ R^1 R^2 R^3 R^4$ et $P^+ R^1 R^2 R^3 R^4$, $R^1$ à $R^4$ ayant la signification donnée en tête du présent brevet. La nature précise du cation phosphonium (ou ammonium) quaternaire n'est pas fondamentale. Le choix parmi les nombreux cations phosphonium (ou ammonium) quaternaire sera avant tout orienté par des considérations d'ordre pratique telles que l'accessibilité ou la facilité de préparation des carboxylates correspondants et la commodité d'emploi des composés en question. Lorsqu'on souhaitera utiliser un tel carboxylate, on fera appel, pour les diverses raisons précitées, aux dérivés du cation méthyltriphénylphosphonium ou du cation méthyltriéthylammonium, bien qu'en principe tout autre cation de ce type puisse également convenir à la mise en pratique de l'invention.

Par contre, le choix de la nature précise de l'anion carboxylate sera avant tout orienté par la nature des produits visés. En effet, comme le montre le schéma réactionnel 1, il est possible d'obtenir par la mise en œuvre du présent procédé des anhydrides d'acides carboxyliques qui peuvent être soit symétriques (lorsque R et R' sont identiques), soit asymétriques (dans le cas contaire). Or, il est bien connu des praticiens que l'anhydride asymétrique peut être transformé par chauffage pour donner naissance aux deux anhydrides symétriques correspondants, selon le schéma réactionnel:

$$2\,RCO{-}O{-}COR' \rightarrow (RCO)_2O + (R'CO)_2O \quad (2)$$

dans lequel R et R' ont la signification précédemment donnée.

Dans le cadre de l'application particulière du présent procédé à la préparation de l'anhydride acétique, on fera bien évidemment appel, de préférence, aux acétates alcalins ou alcalino-terreux ou aux acétates de lanthane, de cérium ou de thorium, les acétates alcalins se révélant plus particulièrement appropriés.

De manière générale, en dehors de cette application particulière, on fait de préférence appel à des carboxylates alcalins.

La concentration du carboxylate de formule III dans le solvant aprotique est en général comprise entre 0,1 et 5 mol/l, et pour une bonne mise en œuvre du présent procédé entre 0,25 et 3 mol/l.

Selon le présent procédé, on met donc en contact, dans une phase liquide pratiquement anhydre renfermant un solvant aprotique, un composé de formule II, un carboxylate de formule III et du monoxyde de carbone. On utilise, de préférence, du monoxyde de carbone sous forme essentiellement pure, tel qu'il se présente dans le commerce. Toutefois, la présence d'impuretés telles que du dioxyde de carbone, de l'oxygène, du méthane et de l'azote peut être tolérée. La présence d'hydrogène n'est pas nuisible, même en des proportions relativement importantes n'excédant toutefois pas 50% molaire dans le mélange gazeux.

Comme le montre le schéma réactionnel 1, il faut théoriquement mettre en œuvre 1/n mol de carboxylate de formule III et 1 mol de monoxyde de carbone par mole de composé de formule II pour produire 1 mol d'anhydride. Bien entendu, les proportions des divers réactifs peuvent s'écarter largement de ces valeurs et on peut utiliser, par exemple, un excès de monoxyde de carbone. De même, il est possible d'utiliser un excès de composé de formule II ou de carboxylate de formule III. En général le rapport molaire entre le composé de formule II et l'anion carboxylate ($R'COO^-$) est compris entre 0,5 et 2 et, pour une bonne mise en œuvre de l'invention, il sera proche du rapport stœchiométrique 1.

La réaction en cause se déroule en présence d'un sel de l'hydrure de tétracarbonylcobalt $[HCo(CO)_4]$.

Les sels de l'hydrure de tétracarbonylcobalt peuvent être représentés par la formule:

$$M^{p+}\,[Co(CO)_4]_p^- \qquad (IX)$$

dans laquelle p est un entier égal à 1 ou 2 et $M^{p+}$ est un cation choisi dans le groupe constitué par les cations des métaux alcalins, les cations alcalino-terreux, les cations $N^+ R^1 R^2 R^3 R^4$, les cations $P^+ R^1 R^2 R^3 R^4$, $R^1$ à $R^4$ ayant la signification précédemment donnée, les cations $Ag^+$, $Mn^{2+}$, $Zn^{2+}$, $Co^{2+}$ et le cation $Hg^{2+}$.

La titulaire, sans pour autant vouloir se lier par une quelconque explication, est amenée à penser que l'anion $[Co(CO)_4]^-$ joue un rôle important dans le déroulement du processus réactionnel et que la nature précise du cation $M^{p+}$ n'est pas fondamentale dans le cadre du présent procédé. Le choix parmi les différents cations possibles est avant tout orienté par des considérations d'ordre pratique relatives à la préparation des sels correspondants.

Ces sels peuvent être préparés extemporanément ou *in situ*. Les sels de l'hydrure de tétracarbonylcobalt, qui sont préparés de préférence au moment de leur emploi, sont pour la plupart des composés connus. Diverses méthodes de préparation de ces composés sont décrites dans la littérature. Ainsi, on pourra se reporter utilement à l'article de J.A. Gladysz et coll., paru dans «Inorganic Chemistry», vol. 18, N° 3, 1979,

pp. 553-558, pour la préparation des sels alcalins de l'hydrure de tétracarbonylcobalt (tétracarbonylcobaltates de lithium, de potassium ou de sodium), ou à l'article de D.H. Gibson et coll., paru dans «Journal of Organometallic Chemistry», 206, 1981, pp. C17-C20, pour la préparation des sels d'ammonium quaternaire de l'hydrure de tétracarbonylcobalt. Cette dernière méthode peut être utilisée également pour la préparation des sels de phosphonium quaternaire de l'hydrure de tétracarbonylcobalt (la modification à apporter à la méthode décrite apparaîtra immédiatement à l'homme de métier).

Bien entendu, comme le savent les spécialistes, les sels de l'hydrure de tétracarbonylcobalt peuvent être formés *in situ* dans les conditions réactionnelles à partir de composés du cobalt plus courants et, pour certains, accessibles dans le commerce. En effet, des sels de cobalt (+2) tels que les halogénures de cobalt, l'acétylacétonate de cobalt, l'acétate de cobalt, le carbonate de cobalt basique et le naphténate de cobalt peuvent être réduits en $[Co(CO)_4]^-$, par exemple, avec de la poudre de manganèse et du thiosulfate de sodium ($Na_2S_2O_3$). Le dicobaltoctacarbonyle $Co_2(CO)_8$ peut par ailleurs constituer une source efficace de tétracarbonylcobaltate dans les conditions de la réaction.

Les sels de l'hydrure de tétracarbonylcobalt peuvent être employés à l'état solide ou bien sous la forme de leur solution dans le solvant choisi pour mettre en œuvre la réaction de carbonylation.

Comme indiqué ci-avant, la nature précise du cation $M^{p+}$ du sel de l'hydrure de tétracarbonylcobalt n'est pas critique. A titre d'exemples de sels utilisables dans le cadre du présent procédé, on peut citer le tétracarbonylcobaltate de sodium, le tétracarbonylcobaltate de lithium, le tétracarbonylcobaltate de potassium et le tétracarbonylcobaltate de méthyltriphénylphosphonium.

La quantité de catalyseur à mettre en œuvre n'est pas fondamentale. On peut, par exemple, utiliser 1 atome-gramme de cobalt ou davantage par mole de composé de formule II. Toutefois, pour une bonne mise en œuvre de l'invention, la quantité de catalyseur, exprimée en milliatomes-grammes de cobalt par mole de composé de formule II, sera comprise entre 2 et 1000 et, de préférence, entre 5 et 100.

La température de réaction sera en général comprise entre 0 et 200° C et, de préférence, entre 20 et 150° C. Pour une bonne mise en œuvre du procédé selon l'invention, on choisira une température voisine de la température ambiante (environ 25° C) lorsque l'on souhaitera utiliser comme matière de départ un carboxylate de formule III dont le cation est choisi parmi les cations de formules $N^+ R^1 R^2 R^3 R^4$ et $P^+ R^1 R^2 R^3 R^4$, dans lesquelles $R^1$ à $R^4$ ont la signification donnée précédemment. Par contre, lorsque l'on mettra en œuvre un carboxylate alcalin il sera avantageux d'opérer à une température supérieure à 50° C.

La pression, à la température choisie, peut varier dans de larges limites, de 0,01 à 600 bar, par exemple. Elle sera de préférence comprise entre 0,5 et 100 bar et, de manière avantageuse, entre 1 et 50 bar.

Pour mettre en œuvre le procédé selon l'invention, on peut, par exemple, introduire dans un réacteur approprié le composé de formule III, le sel de l'hydrure de tétracarbonylcobalt, le solvant de la réaction et le monoxyde de carbone, porter le mélange à la température choisie puis, dans un second stade, introduire le carboxylate. Ce mode opératoire est plus particulièrement adapté lorsque le cation du carboxylate est un cation de formule $P^+ R^1 R^2 R^3 R^4$ ou $N^+ R^1 R^2 R^3 R^4$, $R^1$ à $R^4$ ayant la signification précédemment donnée.

Il est également possible d'introduire dans l'appareillage les réactifs, le solvant de la réaction, le catalyseur et du monoxyde de carbone, puis de porter l'ensemble à la température choisie.

En fin de réaction, l'anhydride produit peut être séparé par tout moyen approprié, par exemple par distillation.

Le procédé selon l'invention trouve une application particulière dans la préparation de l'anhydride acétique par réaction de l'iodure de méthyle, d'un acétate alcalin et du monoxyde de carbone. Par la mise en œuvre du présent procédé, il est en effet possible d'obtenir l'anhydride acétique par une réaction de carbonylation à une température inférieure à 100° C et sous une pression totale en température inférieure à 50 bar, en utilisant un catalyseur à base de cobalt.

Les exemples ci-après illustrent l'invention, sans toutefois en limiter le domaine ou l'esprit.

*Exemples:*

*Préparation de l'acétate de méthyltriphénylphosphonium*

Dans un ballon de 50 ml de capacité, sous argon, on introduit 10 mmol d'iodure de méthyltriphénylphosphonium, 10 mmol d'acétate d'argent et 25 ml d'acétonitrile.

On porte le ballon à 50° C au moyen d'un bain-marie; le mélange est maintenu à cette température pendant 3 h. On refroidit alors, puis on filtre pour éliminer l'iodure d'argent qui a précipité. Le filtrat est récupéré. L'analyse par infrarouge de la solution montre l'existence de bandes correspondant à la présence d'acétate de méthyltriphénylphosphonium.

Le solvant est évaporé sous vide. Une pâte brune est obtenue. Cette pâte peut être utilisée telle quelle ou après redissolution dans le solvant choisi.

*Préparation du tétracarbonylcobaltate de lithium*

Dans un ballon de 100 ml, on charge sous argon 40 ml de tétrahydrofuranne sec, 0,72 mmol de dicobaltoctacarbonyle, puis 1,6 ml d'une solution de 1 mol de triéthylborohydrure de lithium dans le tétrahydrofuranne. On agite à 25° C environ pendant 30 min.

Après arrêt du dégagement d'hydrogène, on arrête l'agitation, filtre la solution sous argon et chasse le solvant. Le résidu est ensuite séché sous vide de 0,5 mmHg à 100° C pendant 30 min.

Une partie du solide est repris dans 10 ml d'acétonitrile. L'analyse par infrarouge de la solution montre les bandes caractéristiques de l'anion tétracarbonylcobaltate.

*Préparation du tétracarbonylcobaltate de méthyltriphénylphosphonium*

5 mmol de dicobaltoctacarbonyle sont dissoutes dans 250 ml de chlorure de méthylène. Le filtrat est coulé dans une solution de 250 ml d'eau distillée contenant 60 mmol de borohydrure de sodium et 15 mmol de chlorure de méthyltriphénylphosphonium. La solution est maintenue sous agitation à température ambiante pendant 3 h environ. Après décantation, la phase organique est séparée, lavée 4 fois avec 100 ml d'eau désoxygénée, puis séchée une nuit sur $Na_2SO_4$.

Le solvant est évaporé et le solide résiduel est dissout dans 30 ml de n-butanol vers 100° C et recristallisé.

Les cristaux sont séparés par filtration et lavés avec 30 ml d'hexane. Après séchage, on isole 3,1 g de tétracarbonylcobaltate de méthyltriphénylphosphonium, soit un rendement de 69%.

Dans ce qui suit, on désignera par r le rapport molaire $RX/R'COO^-$ et par RR (%) le nombre de moles d'anhydride formées pour 100 mol de réactif minoritaire chargé.

*Exemple 1:*

Dans un ballon en verre de 50 ml de capacité, on introduit une solution de 0,6 mmol de tétracarbonylcobaltate de lithium $[Li^+Co^-(CO)_4]$ dans 1 ml d'acétonitrile. On ajoute 8 mmol d'iodure de méthyle tout en faisant barboter du monoxyde de carbone dans la solution. Après 15 min d'agitation à la température ambiante, on introduit dans le ballon une solution de 0,8 mmol d'acétate de méthyltriphénylphosphonium dans 2 ml d'acétonitrile, tout en maintenant pendant 15 min le courant de monoxyde de carbone (r = 10).

Après 12 h de réaction à la température ambiante, le mélange réactionnel résultant est analysé par chromatographie en phase gazeuse. Il contient 0,3 mmol d'anhydride acétique, ce qui correspond à un rendement par rapport à l'acétate de méthyltriphénylphosphonium chargé de 38%.

*Exemple 2:*

On reproduit l'exemple 1 ci-avant, en utilisant une solution de 1 mmol de tétracarbonylcobaltate de méthyltriphénylphosphonium, de formule $CH_3P^+(C_6H_5)_3Co^-(CO)_4$, dans 10 ml de glyme (éther diméthylique de l'éthylèneglycol), 10 mmol d'iodure de méthyle et 12 mmol d'acétate de sodium, sous barbotage de monoxyde de carbone (r = 0,83).

Après 15 h de réaction à la température ambiante, l'analyse par chromatographie en phase gazeuse du mélange réactionnel résultant montre qu'il renferme 1,5 mmol d'anhydride acétique, ce qui correspond à un rendement de 15% par rapport à l'iodure de méthyle chargé.

*Exemple 3:*

On réalise un essai en utilisant le mode opératoire décrit pour l'exemple 1, à partir de 29 mmol d'iodure de méthyle, d'une solution de 1 mmol de tétracarbonylcobaltate de méthyltriphénylphosphonium dans 40 ml de N-méthylpyrrolidone-2 et de 23 mmol d'acétate de sodium, sous barbotage de monoxyde de carbone (r = 1,26). Après 4 h de réaction à 60° C, la pression totale en température étant de l'ordre de 1 bar, l'analyse par chromatographie en phase gazeuse du mélange réactionnel résultant montre qu'il renferme 1,4 mmol d'anhydride acétique, ce qui correspond à un rendement de 6% par rapport à l'acétate de sodium chargé.

*Exemple 4:*

On reproduit l'exemple 3 ci-avant, en utilisant 32 mmol d'iodure de méthyle, une solution de 1 mmol de tétracarbonylcobaltate de méthyltriphénylphosphonium dans 40 ml de glyme et 32 mmol d'acétate de sodium, sous barbotage de monoxyde de carbone (r = 1).

On obtient 1,5 mmol d'anhydride acétique, soit un rendement de l'ordre de 5% par rapport à l'un quelconque des réactifs chargés.

*Exemples 5 à 9 - essai témoin a:*

Une série d'essais est réalisée selon le mode opératoire suivant.

Dans un autoclave en titane de 75 ml de capacité, on charge de l'iodure de méthyle, 30,5 mmol d'acétate de sodium, 1 mmol de tétracarbonylcobaltate de méthyltriphénylphosphonium et 30 ml d'un solvant. Après fermeture de l'autoclave, on admet une pression de monoxyde de carbone de 22 bar. L'agitation, par un système de va-et-vient, est mise en route et l'autoclave est porté à 60° C en 20 min environ, au moyen d'un four annulaire. La pression totale en température est alors égale à 25 bar. Elle est maintenue constante et égale à 25 bar par des recharges successives de monoxyde de carbone. Après 2 h de réaction à la température indiquée, l'agitation est arrêtée; l'autoclave est refoidi et dégazé.

Le mélange réactionnel est alors analysé soit par chromatographie en phase gazeuse, soit par potentiométrie. Dans l'exemple 9, les résultats indiqués sont déterminés à partir du nombre de millimoles de monoxyde de carbone absorbées pendant les 2 h de réaction en température.

Les conditions particulières ainsi que les résultats obtenus sont portés dans le tableau I.

Dans la colonne intitulée dosage, on a indiqué le mode d'analyse comme suit:

1: absorption du monoxyde de carbone
2: chromatographie en phase gazeuse
3: potentiométrie

L'essai témoin a, réalisé dans l'acide acétique, n'entre pas dans le cadre de la présente invention; on n'observe aucune absorption du monoxyde de carbone dans cet essai.

*Tableau I*

| Exemple N° | $CH_3I$ (mmol) | r | Solvant | Dosage | $Ac_2O$ | |
|---|---|---|---|---|---|---|
| | | | | | (mmol) | RR (%) |
| 5 | 32 | 1,05 | Glyme | 2 | 23,7 | 78 |
| 6 | 33,9 | 1,11 | NMP* | 2 | 13,8 | 45 |
| 7 | 32 | 1,05 | TMS* | 3 | 23,0 | 75,4 |
| 8 | 32 | 1,05 | Acétonitrile | 2 | 22,5 | 73,7 |
| 9 | 33 | 1,08 | $Ac_2O$* | 1 | 25,0 | 82,0 |
| a | 32,9 | 1,08 | AcOH* | 1 | 0 | 0 |

* NMP = N-méthylpyrrolidone-2
TMS = tétraméthylènesulfone
$Ac_2O$ = anhydride acétique
AcOH = acide acétique

*Exemple 10:*

On reproduit l'exemple 6 ci-avant avec les modifications suivantes dans la charge: $CH_3I$ = 27 mmol; AcONa = 30 mmol (r = 0,9); tétracarbonylcobaltate de méthyltriphénylphosphonium = 3 mmol.

Après fermeture de l'autoclave, on admet une pression de monoxyde de carbone de 45 bar; la pression à la température de 60° C est maintenue à 50 bar.

On obtient 9,4 mmol d'anhydride acétique [RR (%) = 35], toutes conditions égales par ailleurs.

*Exemples 11 à 15:*

Dans un autoclave en Hastelloy B2 de 125 ml de capacité, on réalise une série d'essais sur une charge constituée par de l'iodure de méthyle, de l'acétate de sodium, 50 ml de glyme et 1 mmol de tétracarbonylcobaltate de méthyltriphénylphosphonium, en utilisant un mode opératoire analogue à celui décrit pour les exemples 5 à 9. Les conditions particulières ainsi que les résultats obtenus en 2 h de réaction à 60° C, sous une pression totale en température maintenue à 25 bar par des recharges successives de monoxyde de carbone, sont rassemblés dans le tableau II. L'analyse des mélanges réactionnels résultants est réalisée par chromatographie en phase gazeuse.

*Tableau II*

| Exemple N° | AcONa (mmol) | $CH_3I$ (mmol) | r | Anhydride acétique | |
|---|---|---|---|---|---|
| | | | | (mmol) | RR (%) |
| 11 | 50 | 48,9 | 0,98 | 48 | 98,0 |
| 12 | 50 | 24,1 | 0,48 | 22,7 | 94,2 |
| 13 | 50 | 10,8 | 0,22 | 8,4 | 77,7 |
| 14 | 12,2 | 49,2 | 4,03 | 12,2 | 100,0 |
| 15 | 100 | 48,9 | 0,49 | 44,1 | 90,2 |

*Exemples 16 à 20 - essai témoin b:*

Dans l'autoclave et selon le mode opératoire décrits pour les exemples 11 à 15, on réalise une série d'essais dont les conditions communes sont les suivantes: la charge est constituée par 50 ml de tétraméthylènesulfone, 1 mmol de tétracarbonylcobaltate de méthyltriphénylphosphonium, de l'iodure de méthyle et un acétate métallique dont la nature et la quantité sont précisées dans le tableau III.

Après fermeture de l'autoclave, on admet une pression de monoxyde de carbone de 22 bar. La température de réaction est de 60° C. La pression totale en température est de 25 bar et elle est maintenue à cette valeur par des recharges successives de monoxyde de carbone. La durée de réaction en température est de 2 h.

Les conditions particulières ainsi que les résultats obtenus figurent dans le tableau III, dans lequel la colonne intitulée dosage a la signification indiquée précédemment.

Au cours de l'essai témoin b on n'observe aucune absorption.

*(Tableau en page suivante)*

*Exemples 21 à 24:*

Dans un autoclave en tantale de 125 ml de capacité, on réalise une série d'essais à partir de différents carboxylates de sodium (R'COONa) à raison d'environ 1 mol/l et de divers composés de formule RX, en utilisant 1 mmol de tétracarbonylcobaltate de méthyltriphénylphosphonium et un mode opératoire analogue à celui décrit pour les exemples 16 à 20. La pression en température est maintenue à 50 bar. En fin des essais 21 à 23, on détermine le nombre de millimoles de fonctions anhydrides (−CO−O−CO−) présentes; pour l'essai N° 24, on a dosé le nombre de millimoles d'anhydride acétique contenues par le mélange réactionnel.

*Tableau III*

| Exemple N° | Acétate métallique | | $CH_3I$ (mmol) | r | Dosage | Anhydride acétique | |
|---|---|---|---|---|---|---|---|
| | Nature | (mmol) | | | | (mmol) | RR (%) |
| 16 | $Zn(OAc)_2$ | 50 | 49 | 0,49 | 2 | 1,3 | 2,7 |
| 17* | $Mn(OAc)_2$ | 50 | 48,3 | 0,48 | 2 | 8,0 | 16,6 |
| 18 | $Mg(OAc)_2$ | 50 | 50 | 0,50 | 2 | 7,0 | 14,0 |
| 19* | Na OAc | 50 | 49,1 | 0,98 | 3 | 40,3 | 82,0 |
| 20 | Li OAc | 33 | 48 | 1,45 | 3 | 24,8 | 75,0 |
| b | $Cr(OAc)_3$ | 50 | 49 | 0,33 | 1 | 0 | 0 |

* L'acétate métallique a été préalablement séché sous vide à 100° C.

Les conditions particulières ainsi que les résultats obtenus sont rassemblés dans le tableau IV dans lequel Ph désigne un reste phényle, OTs désigne un reste tosylate et TMS désigne la tétraméthylènesulfone.

La pression admise est la pression de monoxyde de carbone admise avant la mise en température de l'autoclave.

Les numéros indiqués sur la ligne intitulée dosage ont la signification donnée pour les exemples 5 à 9.

*Tableau IV*

| Exemple N° | 21 | 22 | 23 | 24 |
|---|---|---|---|---|
| RX (mmol) | $CH_3I$ (45,9) | $CH_3I$ (45,8) | n-$C_4H_9I$ (46,2) | $CH_3OTs$ (50) |
| R'COONa (mmol) | Ph-$CH_2$-COONa (50) | Ph-COONa (50) | $CH_3$-COONa (50) | $CH_3COONa$ (50) |
| r | 0,92 | 0,92 | 0,92 | 1 |
| Solvant (ml) | Glyme (50) | Glyme (45) | TMS (50) | Glyme (50) |
| Pression admise | 42 bar | 42 bar | 37 bar | 42 bar |
| Température | 80° C | 80° C | 120° C | 83° C |
| Durée en température | 1 h | 2 h | 2 h | 1 h 40' |
| Dosage | 3 | 3 | 3 | 2 |
| Anhydride (mmol) | 23,8 | 40,1 | 28 | 6,4 |
| Anhydride RR (%) | 51,8 | 87,6 | 60,6 | 12,8 |

*Exemple 25:*

Cet exemple illustre l'utilisation d'un catalyseur formé *in situ* à partir de dicobaltoctacarbonyle.

Dans un autoclave en tantale de 125 ml de capacité, on charge 50 ml de tétraméthylènesulfone, 51,1 mmol d'iodure de méthyle, 50 mmol d'acétate de sodium séché au préalable (r = 1,02), 2 mmol de dicobaltoctacarbonyle et 3 mmol d'iodure de sodium. On admet une pression de monoxyde de carbone de 23 bar. L'agitation est mise en route et l'autoclave est porté à 65° C, en 20 min environ. La pression totale en température est maintenue constante et égale à 25 bar par des recharges successives de monoxyde de carbone. Après 1 h de réaction en température, l'agitation est arrêtée; l'autoclave est refroidi et dégazé.

L'analyse par chromatographie en phase gazeuse du mélange réactionnel résultant montre qu'il contient 39,4 mmol d'anhydride acétique, soit un rendement par rapport à l'iodure de méthyle chargé de 79%.

*Exemples 26 à 28 - essai témoin c:*

Dans un autoclave de 125 ml en Hastelloy B2 (désigné par HB2 dans le tableau V) ou en tantale (désigné par Ta dans ce tableau), on réalise une série d'essais à 70° C, sous une pression en température maintenue constante et égale à 41 bar par des recharges successives de monoxyde de carbone, sur une charge constituée d'iodure de méthyle, d'un acétate métallique dont la nature et la quantité sont précisées dans le tableau V, de

50 ml de N-méthylpyrrolidone et de dicobaltoctacarbonyle.

Les conditions particulières ainsi que les résultats obtenus au moyen d'une analyse par chromatographie en phase gazeuse sont rassemblés dans le tableau V.

*Exemples 29 et 30:*

On a répété les exemples 22 et 23 mais en remplaçant le tétracarbonylcobaltate de méthyltriphénylphosphonium par le dicobaltoctacarbonyle et en maintenant la pression en température à 50 bar (exemple 29) et 80 bar (exemple 30). En fin d'essai, on a dosé par potentiométrie les fonctions anhydrides présentes et identifié et dosé les divers anhydrides formés par résonance magnétique nucléaire du proton.

Les conditions particulières et les résultats obtenus sont consignés dans le tableau VI dans lequel PL désigne un reste phényle et la pression admise est la pression de monoxyde de carbone avant la mise en température de l'autoclave.

*Tableau V*

| Exemple N° | Autoclave | Acétate métallique | | $CH_3I$ (mmol) | r | Durée (min) | Anhydride acétique | |
|---|---|---|---|---|---|---|---|---|
| | | Nature | (mmol) | | | | (mmol) | RR (%) |
| 26 | HB2 | $La(OAc)_3$ | 17 | 49,8 | 0,97 | 120 | 36 | 72 |
| 27 | Ta | $Th(OAc)_4$ | 18,9 | 46,5 | 0,61 | 140 | 42,8 | 92 |
| 28 | Ta | $Zn(OAc)_2$ | 40 | 44,1 | 0,55 | 30 | 30,9 | 70 |
| c | HB2 | $Cr(OAc)_3$ | 17 | 49,3 | 0,97 | 70 | 0 | 0 |

*Tableau VI*

| Exemples | 29 | 30 |
|---|---|---|
| RX (mmol) | $CH_3I$ (49) | n-$C_4H_9I$ (48) |
| R'COONa (mmol) | PhCOONa (50) | $CH_3COONa$ (50) |
| r | 0,98 | 0,96 |
| Solvant (ml) | Glyme (45) | Glyme (45) |
| P admise | 42 bar | 59 bar |
| O | 80° C | 120° C |
| Durée en température | 35 min | 45 min |
| Dosage RMN du proton | $PhCOOCOCH_3$ = 50%<br>$CH_3COOCOCH_3$ = 25%<br>PhCOOCOPh = 25% | $nC_4H_9COOCOCH_3$ = 50%<br>$CH_3COOCOCH_3$ = 25%<br>$(nC_4H_9CO)_2O$ = 25% |
| Anhydride (mmol) | 24 | 38 |
| Anhydride RR (%) | 49 | 79 |

## Revendications

1. Procédé de préparation d'anhydrides d'acides carboxyliques de formule:

$$RCO-O-COR' \qquad (I)$$

dans laquelle R est un radical alkyle linéaire ayant au maximum 4 atomes de carbone et R' est un radical linéaire ou ramifié ayant de 1 à 12 atomes de carbone, un radical phényle ($C_6H_5-$), un radical $C_6H_5-C_xH_{2x}-$ ou un radical $C_xH_{2x+1}-C_6H_4-$, x étant un entier compris entre 1 et 6, R et R' pouvant par ailleurs être identiques ou différents, par réaction, dans une phase liquide pratiquement anhydre renfermant un solvant organique aprotique

a) d'un composé de formule:

$$RX \qquad (II)$$

dans laquelle R a la signification précédente et X représente un atome d'iode ou un groupement $R''-SO_3-$, R'' étant un radical alkyle, aryle ou aralkyle ayant au maximum 10 atomes de carbone,

b) d'un carboxylate de formule:

$$(R'COO^-)_nA^{n+} \qquad (III)$$

dans laquelle R' a la signification précédente, n est un entier égal à 1, 2, 3 ou 4 et $A^{n+}$ est un cation choisi dans le groupe constitué par les cations des métaux alcalins, les cations des métaux alcalino-terreux, les cations $N^+ R^1 R^2 R^3 R^4$ et les cations $P^+ R^1 R^2 R^3 R^4$ dans lesquels $R^1$ à $R^4$, identiques ou différents, ont la signification donnée précédemment pour R', $R^1$ à $R^4$ pouvant en outre être choisis parmi les radicaux cycloalkyles comportant de 3 à 8 atomes de carbone, le cation $Mn^{++}$, le cation $Zn^{++}$, les cations des métaux du groupe des lanthanides et les cations des métaux du groupe des actinides, et

c) du monoxyde de carbone,

en présence d'un sel de l'hydrure de tétracarbonylcobalt, à une température comprise entre 0 et 200° C, sous une pression inférieure ou égale à 600 bar.

2. Procédé selon la revendication 1, caractérisé en ce que le solvant aprotique est un anhydride

d'acide carboxylique de formule (I), correspondant, le cas échéant, à l'anhydride produit.

3. Procédé selon la revendication 1, caractérisé en ce que le solvant aprotique est choisi parmi les éthers alkyliques de polyéthylèneglycols de formule:

$$R^5-O-(CH_2-CH_2-O)_y-R^6 \qquad (IV)$$

dans laquelle $R^5$ et $R^6$, identiques ou différents, ont la signification donnée pour R dans la revendication 1, y étant un entier compris entre 1 et 8.

4. Procédé selon la revendication 1, caractérisé en ce que le solvant aprotique est choisi parmi les sulfones de formule:

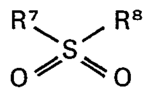
(V)

dans laquelle $R^7$ et $R^8$, identiques ou différents, ont la signification donnée pour R' dans la revendication 1; $R^7$ et $R^8$ peuvent en outre former ensemble un radical unique divalent – alkylène ou alcénylène – comportant de 3 à 6 atomes de carbone (par exemple un radical tétraméthylène ou hexaméthylène) et, le cas échéant, 1 ou 2 doubles liaisons éthyléniques, ledit radical pouvant comporter 1 à 3 substituants alkyles ayant de 1 à 4 atomes de carbone.

5. Procédé selon la revendication 4, caractérisé en ce que $R^7$ et $R^8$ sont identiques et choisis parmi les radicaux alkyles linéaires.

6. Procédé selon l'une des revendications 1 ou 4, caractérisé en ce que le solvant aprotique est choisi parmi la tétraméthylènesulfone, la méthyl-3-tétraméthylènesulfone, la diméthyl-2,4-tétraméthylènesulfone et leurs mélanges.

7. Procédé selon la revendication 1, caractérisé en ce que le solvant aprotique est choisi parmi les amides d'acides carboxyliques de formule:

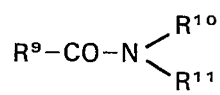
(VI)

dans laquelle $R^9$, $R^{10}$ et $R^{11}$, identiques ou différents, ont la signification donnée pour R' dans la revendication 1; deux des radicaux $R^9$, $R^{10}$, $R^{11}$ peuvent par ailleurs constituer ensemble un radical unique divalent $-(CH_2)_z-$, z étant un entier compris entre 3 et 12; $R^9$ peut en outre représenter un atome d'hydrogène ou un radical de formule:

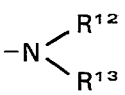
(VII)

dans laquelle $R^{12}$ et $R^{13}$, identiques ou différents, représentent des radicaux alkyles ayant au maximum 4 atomes de carbone.

8. Procédé selon l'une des revendications 1 ou 7, caractérisé en ce que le solvant aprotique est la N-méthylpyrrolidone-2.

9. Procédé selon la revendication 1, caractérisé en ce que le solvant aprotique est l'acétonitrile.

10. Procédé selon l'une des revendications précédentes, caractérisé en ce que le carboxylate de formule (III) est un carboxylate alcalin.

11. Procédé selon l'une des revendications précédentes, caractérisé en ce que le carboxylate de formule (III) est un acétate.

12. Procédé selon l'une des revendications précédentes, caractérisé en ce que la concentration du carboxylate de formule (III) dans le solvant aprotique est comprise entre 0,1 et 5 mol/l.

13. Procédé selon l'une des revendications précédentes, caractérisé en ce que le rapport molaire entre le composé de formule (II) et l'anion carboxylate ($R'COO^-$) est compris entre 0,5 et 2.

14. Procédé selon l'une des revendications précédentes, caractérisé en ce que le catalyseur utilisé est choisi parmi le tétracarbonylcobaltate de lithium, le tétracarbonylcobaltate de sodium et le tétracarbonylcobaltate de méthyltriphénylphosphonium.

15. Procédé selon l'une des revendications précédentes, caractérisé en ce que le catalyseur est formé *in situ* à partir de dicobaltoctacarbonyle.

16. Procédé selon l'une des revendications précédentes, caractérisé en ce que le composé de formule (II) utilisé est l'iodure de méthyle.

17. Procédé selon l'une des revendications précédentes, caractérisé en ce que la température de réaction est comprise entre 20 et 150° C.

18. Procédé selon l'une des revendications précédentes, caractérisé en ce que la pression en température est comprise entre 1 et 100 bar.

## Claims

1. Process for the preparation of carboxylic acid anhydrides of the formula:

$$RCO-O-COR' \qquad (I)$$

in which R is a linear radical having at most 4 carbon atoms and R' is a linear or branched alkyl radical having from 1 to 12 carbon atoms, a phenyl radical ($C_6H_5-$), a radical $C_6H_5-C_xH_{2x}-$ or a radical $C_xH_{2x+1}-C_6H_4-$, x being an integer between 1 and 6, it also being possible for R and R' to be identical or different, by reacting the following in a virtually anhydrous, liquid phase containing an aprotic organic solvent:

(*a*) a compound of the formula:

$$RX \qquad (II)$$

in which R has the above meaning and X represents an iodine atom or a group $R''-SO_3-$, R'' being an alkyl, aryl ou aralkyl radical having at most 10 carbon atoms,

(*b*) a carboxylate of the formula:

$$(R'COO^-)_nA^{n+} \qquad (III)$$

in which R' has the above meaning, n is an integer equal to 1, 2, 3 or 4 and $A^{n+}$ is a cation chosen from the group comprising alkali metal cations, alkaline earth metal cations, the cations $N^+R^1R^2R^3R^4$ and the cations $P^+R^1R^2R^3R^4$, in which $R^1$ to $R^4$, which are identical or different, have the meaning previously given for R', it also being possible for $R^1$ to $R^4$ to be chosen from amongst cycloalkyl radicals containing from 3 to 8 carbon atoms, the

cation $Mn^{++}$, the cation $Zn^{++}$, the cations of metals from the lanthanide group and the cations of metals from the actinide group, and

(*c*) carbon monoxide,

in the presence of a salt of cobalt tetracarbonyl hydride, at a temperature of between 0 and 200° C, under a pressure which is less than or equal to 600 bar.

2. Process according to Claim 1, characterised in that the aprotic solvent is a carboxylic acid anhydride of the Formula (I), corresponding, if appropriate, to the anhydride produced.

3. Process according to Claim 1, characterised in that the aprotic solvent is chosen from amongst the polyethylene glycol alkyl ethers of the formula:

$$R^5-O-(CH_2-CH_2-O)_y-R^6 \qquad (IV)$$

in which $R^5$ and $R^6$, which are identical or different, have the meaning given for R in Claim 1, y being an integer between 1 and 8.

4. Process according to Claim 1, characterised in that the aprotic solvent is chosen from amongst the sulphones of the formula:

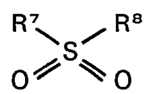

(V)

in which $R^7$ and $R^8$, which are identical or different, have the meaning given for R' in Claim 1; $R^7$ and $R^8$ can also together form a single divalent alkylene or alkenylene radical containing from 3 to 6 carbon atoms (for example a tetramethylene or hexamethylene radical) and, if appropriate, 1 or 2 ethylenic double bonds, it being possible for the said radical to contain 1 to 3 alkyl substituents having from 1 to 4 carbon atoms.

5. Process according to Claim 4, characterised in that $R^7$ and $R^8$ are identical and chosen from amongst linear alkyl radicals.

6. Process according to one of Claims 1 or 4, characterised in that the aprotic solvent is chosen from amongst tetramethylenesulphone, 3-methyltetramethylenesulphone, 2,4-dimethyltetramethylenesulphone and mixtures thereof.

7. Process according to Claim 1, characterised in that the aprotic solvent is chosen from amongst the carboxylic acid amides of the formula:

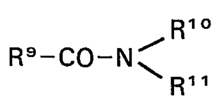

(VI)

in which $R^9$, $R^{10}$ and $R^{11}$, which are identical or different, have the meaning given for R' in Claim 1; two of the radicals $R^9$, $R^{10}$, $R^{11}$ can also together form a single divalent radical $-(CH_2)_z-$, z being an integer between 3 and 12; $R^9$ can also represent a hydrogen atom or a radical of the formula:

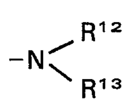

(VII)

in which $R^{12}$ and $R^{13}$, which are identical or different, represent alkyl radicals having at most 4 carbon atoms.

8. Process according to one of Claims 1 or 7, characterised in that the aprotic solvent is N-methylpyrrolidin-2-one.

9. Process according to Claim 1, characterised in that the aprotic solvent is acetonitrile.

10. Process according to one of the preceding claims, characterised in that the carboxylate of the formula (III) is an alkali metal carboxylate.

11. Process according to one of the preceding claims, characterised in that the carboxylate of the formula (III) is an acetate.

12. Process according to one of the preceding claims, characterised in that the concentration of the carboxylate of the formula (III) in the aprotic solvent is between 0.1 and 5 mol/l.

13. Process according to one of the preceding claims, characterised in that the molar ratio of the compound of the formula (II) to the carboxylate anion ($R'COO^-$) is between 0.5 and 2.

14. Process according to one of the preceding claims, characterised in that the catalyst used is chosen from amongst lithium tetracarbonylcobaltate, sodium tetracarbonylcobaltate, and methyltriphenylphosphonium tetracarbonylcobaltate.

15. Process according to one of the preceding claims, characterised in that the catalyst is formed *in situ* from dicobaltoctacarbonyl.

16. Process according to one of the preceding claims, characterised in that the compound of the formula (II) used is methyl iodide.

17. Process according to one of the preceding claims, characterised in that the reaction temperature is between 20 and 150° C.

18. Process according to one of the preceding claims, characterised in that the pressure at the reaction temperature is between 1 and 100 bar.

## Patentansprüche

1. Verfahren zur Herstellung von Carbonsäureanhydriden der Formel:

$$RCO-O-COR' \qquad (I)$$

in der R eine lineare Alkylgruppe mit maximal 4 Kohlenstoffatomen und R' eine lineare oder verzweigte Alkylgruppe mit 1 bis 12 Kohlenstoffatomen, eine Phenylgruppe ($C_6H_5-$), eine Gruppe $C_6H_5-C_xH_{2x}-$ oder eine Gruppe $C_xH_{2x+1}-C_6H_4-$ ist, x eine ganze Zahl von 1 bis 6 beudeutet, wobei R und R' gleich oder verschieden sein können, durch Umsetzung in praktisch wasserfreier flüssiger Phase, die ein aprotisches organisches Lösungsmittel enthält:

a) einer Verbindung der Formel:

$$RX \qquad (II)$$

in der R die oben angegebene Bedeutung hat und X ein Iodatom oder eine Gruppe $R''-SO_3-$ bedeutet, wobei R'' eine Alkyl-, Aryl- oder Aralkylgruppe mit maximal 10 Kohlenstoffatomen ist, mit

b) einem Carboxylat der Formel:

$$(R'COO^-)_nA^{n+} \qquad (III)$$

in der R' die oben angegebene Bedeutung hat, n eine ganze Zahl gleich 1, 2, 3 oder 4 ist und $A^{n+}$ ein

Kation bedeutet, das ausgewählt ist aus der Gruppe, bestehend aus den Kationen der Alkalimetalle, der Erdalkalimetalle, $N^+R^1R^2R^3R^4$ und $P^+R^1R^2R^3R^4$, wobei $R^1$ bis $R^4$, gleich oder verschieden, die oben für R' angegebene Bedeutung haben und $R^1$ bis $R^4$ ausserdem Cycloalkylgruppen mit 3 bis 8 Kohlenstoffatomen bedeuten können, $Mn^{++}$, $Zn^{++}$ sowie Kationen der Metalle der Gruppe der Lanthaniden und der Gruppe der Actiniden, und

c) Kohlenmonoxid

in Gegenwart eines Salzes von Tetracarbonylcobalthydrid bei einer Temperatur von 0 bis 200° C unter einem Druck von 600 bar oder darunter.

2. Verfahren nach Anspruch 1, dadurch gekennzeichnet, dass das aprotische Lösungsmittel ein Carbonsäureanhydrid der Formel (I) ist, das ggf. dem erzeugten Anhydrid entspricht.

3. Verfahren nach Anspruch 1, dadurch gekennzeichnet, dass das aprotische Lösungsmittel ein Polyethylenglykolalkylether der Formel:

$$R^5-O-(CH_2-CH_2-O)_y-R^6 \qquad (IV)$$

ist, in der $R^5$ und $R^6$ gleich oder verschieden sind und die für R in Anspruch 1 angegebene Bedeutung haben und y eine ganze Zahl von 1 bis 8 ist.

4. Verfahren nach Anspruch 1, dadurch gekennzeichnet, dass das aprotische Lösungsmittel ein Sulfon der Formel:

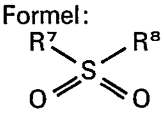 (V)

ist, in der $R^7$ und $R^8$ gleich oder verschieden sind und die für R' in Anspruch 1 angegebene Bedeutung haben, $R^7$ und $R^8$ weiterhin eine einzige zweiwertige Gruppe – Alkylen- oder Alkenylengruppe – mit 3 bis 6 Kohlenstoffatomen (beispielsweise eine Tetramethylen- oder Hexamethylengruppe) und ggf. eine oder zwei ethylenische Doppelbindungen sein können, die 1 bis 3 Alkylsubstituenten mit 1 bis 4 Kohlenstoffatomen aufweisen können.

5. Verfahren nach Anspruch 4, dadurch gekennzeichnet, dass $R^7$ und $R^8$ identisch und unter den linearen Alkylgruppen ausgewählt sind.

6. Verfahren nach einem der Ansprüche 1 oder 4, dadurch gekennzeichnet, dass das aprotische Lösungsmittel unter Tetramethylensulfon, 3-Methyltetramethylensulfon, 2,4-Dimethyltetramethylensulfon und deren Gemischen ausgewählt ist.

7. Verfahren nach Anspruch 1, dadurch gekennzeichnet, dass das aprotische Lösungsmittel unter den Carbonsäureamiden der Formel:

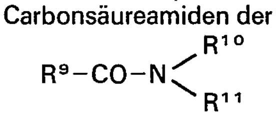 (VI)

ausgewählt ist, in der $R^9$, $R^{10}$ und $R^{11}$ gleich oder verschieden sind und die für R' in Anspruch 1 angegebene Bedeutung haben, zwei der Gruppen $R^9$, $R^{10}$, $R^{11}$ ausserdem zusammen eine einzige zweiwertige Gruppe $-(CH_2)_z-$ sein können, in der z eine ganze Zahl von 3 bis 12 ist, $R^9$ darüber hinaus ein Wasserstoffatom oder eine Gruppe der Formel:

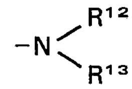 (VII)

sein kann, in der $R^{12}$ und $R^{13}$, gleich oder verschieden, Alkylgruppen mit maximal 4 Kohlenstoffatomen bedeuten.

8. Verfahren nach einem der Ansprüche 1 oder 7, dadurch gekennzeichnet, dass das aprotische Lösungsmittel N-Methylpyrrolidon-2 ist.

9. Verfahren nach Anspruch 1, dadurch gekennzeichnet, dass das aprotische Lösungsmittel Acetonitril ist.

10. Verfahren nach einem der vorangehenden Ansprüche, dadurch gekennzeichnet, dass das Carboxylat der Formel (III) ein Alkalicarboxylat ist.

11. Verfahren nach einem der vorangehenden Ansprüche, dadurch gekennzeichnet, dass das Carboxylat der Formel (III) ein Acetat ist.

12. Verfahren nach einem der vorangehenden Ansprüche, dadurch gekennzeichnet, dass die Konzentration des Carboxylats der Formel (III) im aprotischen Lösungsmittel 0,1 bis 5 mol/l beträgt.

13. Verfahren nach einem der vorangehenden Ansprüche, dadurch gekennzeichnet, dass das Molverhältnis von Verbindung der Formel (II) zu Carboxylatanion ($R'COO^-$) 0,5 bis 2 beträgt.

14. Verfahren nach einem der vorangehenden Ansprüche, dadurch gekennzeichnet, dass als Katalysator Lithiumtetracarbonylcobaltat, Natriumtetracarbonylcobaltat oder Methyltriphenylphosphoniumtetracarbonylcobaltat gewählt wird.

15. Verfahren nach einem der vorangehenden Ansprüche, dadurch gekennzeichnet, dass der Katalysator ausgehend von Dicobaltoctacarbonyl *in situ* gebildet wird.

16. Verfahren nach einem der vorangehenden Ansprüche, dadurch gekennzeichnet, dass als Verbindung der Formel (II) Methyliodid eingesetzt wird.

17. Verfahren nach einem der vorangehenden Ansprüche, dadurch gekennzeichnet, dass die Reaktionstemperatur 20 bis 150° C beträgt.

18. Verfahren nach einem der vorangehenden Ansprüche, dadurch gekennzeichnet, dass der Druck bei der angewandten Temperatur 1 bis 100 bar beträgt.